# EUROPEAN PATENT APPLICATION

(11) **EP 4 201 211 A1**
(43) Date of publication of application: **28.06.2023**
(21) Application number: 21217173.0
(22) Date of filing: 22.12.2021
(51) Int. Cl.: A21D 8/04, A23L 7/104, C12N 1/20

(54) **PASTA SOURDOUGH CULTURES AND METHODS OF MAKING SAME**

(71) Applicant: Lettrari, Silvio Umberto, Kaslo BC V0G1M0 (CA)
(72) Inventor: Lettrari, Silvio Umberto, Kaslo BC V0G1M0 (CA)
(74) Representative: Forresters IP LLP

(57) **Abstract**

The present disclosure relates to the preparation and maintenance of sourdough cultures that can be used to prepare pasta. The disclosure describes the identification of specific fermentation conditions that reliably maintain lactate-utilizing lactobacilli as dominant members of the microbiota by traditional methods. A crucial difference between known processes and the inventive process is that the conditions of the inventive fermentation process naturally select for several species of microorganisms, including *Levilactobacillus koreensis* and *Levilactobacillus parabrevis.* Specifically, the cold fermentation favors growth of these organisms and thereby produces more acetic acid than lactic acid in the sourdough.

## Description

### FIELD OF THE INVENTION

The present invention relates generally to the field of sourdough cultures, specifically to sourdough cultures that are capable of converting lactic acid to acetic acid and 1,2-propanediol, more specifically such sourdough cultures and fermented sourdough compositions for use in the preparation of pasta.

### BACKGROUND

Foods derived from cereal grains, such as bread and pasta are key constituents of the diet and represent staple foods in many countries. Lowering the glycemic index of staple foods is of great interest as such foods are generally consumed in large quantities, have high caloric density and are implicated in the rising obesity epidemic *(*Am J Clin Nutr. (2002) 76:266-731). Furthermore, there is growing consumer interest in foods with improved nutritional and functional properties. Sourdough fermentation is an ancient technology traditionally used to leaven bread (Int J Food Microbiol. (2019) 302:15- 23). Sourdough cultures are typically dominated by lactic acid bacteria in symbiotic combination with yeasts *(*Trends Food Sci Technol. (2005) 16:43-56, and Trends Food Sci Technol. (2017) 68:152-9).

Lactic acid bacteria are known to make species- or strain specific contributions to product quality but sourdough fermentation, typically at room temperature, generally increases acidity through production of lactic and acetic acids *(*Food Microbiol. (2014) 37:2-10). Sourdough fermentation increases protein digestibility, total soluble fiber content, reduces the glycemic index of food and improves the bioavailability of minerals *(*Foods. (2019) 8:1-21, and Crit Rev Food Sci Nutr. (2019) 60:2158-73).

In addition, fermentation is considered to be a sustainable and effective method to make whole grain and fiber-rich products more palatable while also improving the functional and nutritional value of the products (Food Microbiol. (2009) 26:693-9). While traditionally used in bread making, sourdough cultures are becoming more prominent in other novel applications including the use of sourdough in pasta making

Production of food and food ingredients via microbial fermentation is characterized by an intrinsic complexity. The raw material and the final products typically have a complex composition: fermented food is often the result of the metabolic activity of mixed microbial populations, resulting in the production of complex ensemble of primary and secondary metabolites that are responsible for the main and specific features of a certain food or beverage

Starter cultures are an essential component of nearly all commercially produced fermented foods. Simply defined, starter cultures consist of microorganisms that are inoculated directly into food materials in order to bring about desired and predictable changes in the finished product. These changes may include enhanced preservation, improved nutritional value, modified sensory qualities, and increased economic value. Although many fermented foods can be made without a starter culture, the addition of concentrated microorganisms, in the form of a starter culture, provides a basis for insuring that products are manufactured on a consistent schedule, with consistent product qualities.

Fermented foods and beverages have long been manufactured without the use of commercial starter cultures. Traditional methods of production include back-slopping, or using a small amount of the finished specifically preserved product to inoculate a new batch, the use of microorganisms found naturally on the product, and the use of special containers that allow for the survival of the starter culture microorganisms within cracks and pores.

Food Microbiol (2010) 27:390-395 - discloses cooperative metabolism of lactobacilli in silage fermentation converts lactate to propionate. This study aimed to determine whether propionate production by *Lentilactobacillus buchneri* and *Lentilactobacillus diolivorans* can be applied for bread preservation. Propionate formation was observed in co-fermentation with *Ln. buchneri* and *Ln. diolivorans* in modified MRS broth as well as sourdough with low, medium and high ash contents. 48 mM of propionate was formed in sourdough with medium ash content, but only 9 and 28 mM propionate were formed in sourdoughs prepared from white wheat flour or whole wheat flour, respectively. Acetate levels were comparable in all three sourdoughs and ranged from 160 to 175 mM. Sourdough fermented with *Ln. buchneri* and *Ln. diolivorans* was used in breadmaking and its effect on fungal spoilage was compared to traditional sourdough or propionate addition to straight doughs. Bread slices were inoculated with *Aspergillus clavatus, Cladosporium spp., Mortierella spp.* or *Penicillium roquefortii.* The use of 20% experimental sourdough inhibited growth of three of the four moulds for more than 12 days. The use of 10% experimental sourdough deferred growth of two moulds by one day. Bread from traditional sourdough with added acetate had less effect in inhibiting mould growth. In conclusion, cofermentation with *Ln. buchneri* and *Ln. diolivorans* represents a process to increase antifungal capacities of bread.

Current Opinion in Food Science 2015, 2:106-117 - discloses sensory properties, shelf life, and safety of a majority of fermented foods are determined by the metabolic activity of food fermenting lactic acid bacteria. This communication reviews major metabolic routes of lactic acid bacteria, and indicates how metabolism is influenced by the environmental conditions or manipulated for improved control of food fermentations. Emphasis is placed on homofermentative and heterofermentative metabolism of carbohydrates, organic acids, and the conversion of amino acids with major impact on food safety and quality. In addition to the role of lactic metabolism in food fermentations, their implications for food spoilage are discussed.

App and Environ Microbiol (2015) 81:7233-7243 - discloses a study relating to lactobacilli use in food, feed, and health applications. The taxonomy of lactobacilli, however, was confounded by the apparent lack of physiological markers for phylogenetic groups of lactobacilli and the unclear relationships between the diverse phylogenetic groups. This study used the core and pan-genomes of 174 type strains of lactobacilli to establish phylogenetic relationships and to identify metabolic properties differentiating phylogenetic groups. The core genome phylogenetic tree separated homofermentative lactobacilli and pediococci from heterofermentative lactobacilli. Aldolase and phosphofructokinase were generally present in homofermentative but not in heterofermentative lactobacilli; a two-domain alcohol dehydrogenase and mannitol dehydrogenase were present in most heterofermentative lactobacilli but absent in most homofermentative organisms. Other genes were predominantly present in homofermentative lactobacilli (pyruvate formate lyase) or heterofermentative lactobacilli (lactaldehyde dehydrogenase and glycerol dehydratase). Cluster analysis of the phylogenomic tree and the average nucleotide identity grouped the genus *Lactobacillus* sensu lato into 24 phylogenetic groups, including pediococci, with stable intra- and intergroup relationships. Individual groups may be differentiated by characteristic metabolic properties. The link between phylogeny and physiology that is proposed in this study facilitates future studies on the ecology, physiology, and industrial applications of lactobacilli.

Trends in Food Science & Technology (2008) 19:513-521 describe a commercial probiotic mixture in combination with addition of protease to reduce the gluten level in wheat sourdoughs to less than 200 ppm. The strains are largely unrelated to sourdoughs (only *Lacticaseibacillus casei, Lactobacillus helveticus* and *Lactiplantibacillus plantarum* occur in specific sourdoughs. Sourdough lactobacilli are rarely proteolytic. Most of these do not have extracellular proteases and proteins do not enter the bacterial cytoplasm. If strains have extracellular proteases, as some strains of *Lactobacillus helveticus* do, these are repressed ("shut down") in conditions that provide an ample supply of peptides and amino acids, which includes each and every wheat sourdough. This publication is distinguishable from the present disclosure in that the claimed sourdough does not includes protease, and does not aim to reduce of gluten levels in wheat doughs below 200 ppm.

CA 2759532 - Ernst Boecker - discloses method for producing sourdough and baked goods having an extended shelf life, wherein biological preservatives are augmented in the dough or the baked goods by co-fermenting selected lactobacilli. The method involves room temperature co-fermentation two strains of lactic acid bacteria, wherein one of the strains is *Lentilactobacillus diolivorans.* This application relates to the inoculation of the sourdough with selected strains, however does not disclose the specific fermentation conditions that select for lactate to acetate plus propanediol without using pure cultures.

US20200214301 - Ernst Boecker - discloses a well-tolerated flour composition. The invention relates to the field of food production, in particular the provision of flour mixtures for the production of bakery products, pasta and bread, which are characterized by a reduced amylase trypsin inhibitors (ATI) content and can nevertheless be processed into doughs which meet the technical and rheological requirements of a wheat dough. The present invention concerns the field of food production, in particular the production and supply of bakery products and breads which are digestible despite known food intolerances. The invention is limited to embodiments wherein the ATI-reduced flour mixture is mixed with sourdough, sourdough concentrate, baking additives and/or leavening agents. Moreover, the sourdoughs or sourdough concentrates used are limited to containing one or more strains or pure breeding strains from the group of microorganisms containing *Lv. acidifarinae, L. acidophilus, Co. alimentarius, L. amylovorus, Lv. brevis, Ln. buchneri, Lm. fermentum* (previously: *L. cellobiosus), Lm. coleohominis, Sc.. collinoides, L. crispatus, Co. crustorum, Lt. curvatus, L. delbrueckki, Co. farciminis, Fl. fructivorans* (prev: *L. homohiochii), Lm. frumenti, L. gallinarum, L. gasseri, Lv. hammesii, L. helveticus, Ln. hilgardii, L. johnsonii, Ln. kefiri, Co. kimchii, Ap. kunkeei, Fl. lindenri, Lq. mali, Co. mindensis, Lm. mucosae, Lq. nagelii, Co. nantensis, Co. namurensis, Co. nodensis, Lm. oris, Lm. panis, Co. paralimentarius, Ln. parabuchneri, Lc. paracasei, Lp. pentosus, Sl. perolens, Lp. plantarum, Lm. pontis, Lm. reuteri, Ff. rossiae, Lr. sakei, Fl. sanfranciscensis, Lm. secaliphilus, Ff. siliginis, Lv. spicheri, L. vaginalis, Lv. zymae,* and others *Lactococcus lactis, Leuconostoc citreum, L. gelidum, Lc. mesenteroides, Pediococcus acidilactici, Pediococcus damnosus, P. parvulus, P. pentosaceus, Weissella ciboria, Weissella confusa, Weissella kandleri, Weissella paramesenteroides, Weissella viridescens, Kazachstania milleri* (prev. *Candida milleri* or *Candida humilis), Kazachstania exigua, Saccharomyces cerevisiae, Debaryomyces hansenii, Dekkera bruxellensis, Kazachstania unispora, Kluyveromyces lactis, S. bayanus, Saccharomyces pastorianus, Torulaspora delbrueckii, T. pretoriensis, Wickerhamomyces anomalus, Pichia anomala, Hansenula anomala, Pichia kudriavzevii, Issatschenkia orientalis, Candida krusei* and mixtures thereof.

US20080131556 - VSL Pharmaceuticals Inc - discloses a mixture of at least 6 species of lactic acid bacteria and/or bifidobacteria in the manufacture of a sourdough. The mixture of at least 6 species of lactic acid bacteria and/or Bifidobacteria is disclosed for use in bakery and medical field. The preferred mixture comprises *Streptococcus thermophilus, Bifidobacterium infantis, Bifidobacterium longum, Bifidobacterium breve, Lactobacillus acidophilus, Lactiplantibacillus plantarum, Lacticaseibacillus casei, Lactobacillus delbrueckii subsp. bulgaricus.* The mixture is useful for a sourdough, a leavening composition. Baked goods and other food products obtained therefrom are disclosed. These goods have low or no gluten content and are suitable for the integration of the diet of a subject suffering from celiac disease, for decreasing the risk of allergies due to wheat flour albumins and globulins, for the treatment of schizophrenic symptoms, in the preparation of products for enteric diet. In preferred embodiments of the invention, the sourdough contains a fungal protease from *Aspergillus oryzae;* activity of 500,000 HUT/g; pH optimum about 3.0 and activity in the range of pH 3.0 to 6.0; temperature optimum about 50 C and activity in the range 25- 60 C; or another protease is an acid stable protease from *Aspergillus niger;* activity of 3,000 SAPU/g; optimum pH 2.0-3.0 and activity in the range of pH 2,0 to 6,0; temperature optimum 50-60 C and activity in the range 30-60 C.

CA2518368 - Lesaffre et Cie - discloses a sourdough, the use thereof and bakery products produced from the same. The invention relates to a method for production of ready-to-use sour doughs, containing homo-fermenting lactic bacteria, whereby said method permits a variation of the fermenting quotient of said doughs. The invention further relates to doughs produced by said method and use of said doughs in breadmaking. The invention also relates to a sourdough leaven ready use, a process for its preparation and its use in breadmaking as a fermentation agent.

WO2006048752 - Ista S.P.A. - discloses a method for controlling and standardizing the production of traditional pasta by using GRAS microorganisms. The invention describes the identification, control and optimisation procedures of the physico-chemical and biological reactions of the production process for traditional pasta; it identifies the most suited natural coadjuvant mixtures and defines the control method of the identified process in a user protocol. The process defines the durum wheat flour mixtures having suitable technological characteristics and develops a reference formulation for traditional pasta making; it identifies the combination of microbial species/strains most suitable for use and characterises it genetically and enzymatically as a function of flour component hydrolytic activity. It defines the parameters of the physical texture and the organoleptic characteristics of pasta dough subjected to a low temperature drying process. Preferred microorganisms for obtaining a useful mixture for achieving the effects described include: Yeasts; *Saccharomyces cerevisiae, Saccharomyces exiguus, Saccharomyces bayanus, Saccharomyces pastorianus, Candida h*umilis, *Candida milleri, Candida holmii, Candida norvegensis, Candida boidinii, Hansenula anomala.* Microbes; *Fructilactobacillus sanfranciscensis* , *Levilactobacillus brevis, Companilactobacillus alimentarius, Lactiplantibacillus plantarum, Limosilactobacillus fermentum, Limosilactobacillus pontis, Pediococcus pentosaceus, Leuconostoc mesenteroides, Weissella confusa, Leuconostoc lactis, Micrococcus spp., Bacillus spp., Enterococcus spp.*

WO2019149839 - Pascal Philibert - discloses a sourdough and process for producing same. Sourdough having a pH of between 3 and 6, a content of acetic acid of less than 2000 ppm, a content of lactic acid of less than 8000 ppm, a content of yeast in log(CFU)/g of between 7 and 9, a content of lactic acid bacteria in log(CFU)/g of between 8 and 10 and a growth measurement at 5 h in cm of greater than 2, which, when used in bakery, gives a bread having a specific volume of the cooked bread at least equal to 3 cm3/g. The invention is a leaven and its method of obtaining, capable of raising, without the addition of yeast, all types of pasta and in an improvement of also fermenting the dough of leavened bread. The leaven can be used to produce all pasta requiring fermentation like bread and all its variants, campaign, cereal, tradition, corn, baguette etc., bread, buns and derivatives, pastries, brioches, croissants, panettone and their variants, pizza dough.

CN102919295 - Univ Dalian - discloses a preparation method of leavened pasta. The invention provides a preparation method of leavened pasta. The method is characterized in that a flour leavening agent contains composite microorganisms from Tibetan kefir. According to the invention, the flour leavening agent containing microorganisms from Tibetan kefir is applied to the preparation of leavened pasta for the first time to obtain the leavened pasta with nutrition and healthcare effect and special flavor, the types of the flavor leavened pasta are increased, and the industrial production brings considerable economic benefits.

CN104010514 - Univ Dalian - discloses methods of making natural sourdough starter for baking bread and methods of making bread using the same. A method of preparing a natural sourdough starter capable of being continuously prepared while allowing bread to maintain uniform quality activities is provided. In particular, a method of preparing a natural sourdough starter for bread using a traditional Nuruk is provided. The method includes mixing purified water with a Songhak Nuruk, putting the resulting mixture into an incubator and separating and filtering a microorganism starter, mixing purified water, wheat flour and rye flour with the microorganism starter, putting the resulting mixture into a fermenter and fermenting the mixture at room temperature, mixing purified water, wheat flour and rye flour with the starter culture broth, putting the resulting mixture into an incubator and fermenting the mixture at a temperature of 11C to 13C, and putting the sourdough starter into a refrigerator and refrigerating the sourdough starter at a temperature of 2C to 4C.

DE202016101614 - Ernst Boecker - discloses gluten-free baked goods. The sourdough contains at least two sourdough pure cultures selected from the group of the microorganisms consisting of *Lp. plantarum, Lm. pontis, Fl. sanfranciscensis, L. crispatus, L. helveticus* (prev. *L. suntoryeus, Le. argentinum, Lm. secaliphilus, Lm. panis, L. amylovorus, Co. paralimentarius, Lm. fermentum, Lc. paracasei, Lm. frumenti, Co. alimentarius, W. cibaria, W. confusa, Kz. milleri* (prev. *C. humilis* or *C*. *milleri), Kz. exiguous* (prev. *S. exiguous), S. minor, S. pastorianus* and *S. fructuum, at* least one treated with heat-moisture and gluten-free flour, and at least one more gluten-free flour, as well at least one more conventional and gluten-free baking ingredient, wherein the composition is a gluten-free baking mix or baked good.

WO2015158960 - Teknologian Tutkimuskeskus Vtt Oy - discloses gluten-free pasta and method for the manufacture of gluten-free pasta. The invention relates to a method comprising the steps of grinding fava beans, subjecting the ground fava beans to fractionation by air classification, separating a fraction having average particle size of about D50 = 20 -100 µm and D90 = 40 -150 µm, and blending gluten-free flour comprising the separated fraction, with water to obtain pasta dough. The invention also relates to gluten-free pasta comprising the fava bean fraction and to the use of the fava bean fraction in the manufacture of gluten-free pasta.

All documents cited herein are incorporated by reference.

None of the above cited documents, alone or in combination satisfy the need for a sourdough that creates specific fermentation conditions that reliably maintain lactate-utilizing lactobacilli as dominant members of the microbiota resulting in a sourdough that can be used to produce sourdough pasta. pasta.

### SUMMARY OF THE INVENTION

It is an object of the invention pasta sourdough cultures and methods of making same.

In accordance with an aspect of the invention there is provided a composition for producing a sourdough, the composition comprising one or more lactobacilli, that catalyze lactate to propanediol and acetate conversion, the lactobacilli selected from the *Levilactobacillus* species.

In accordance with another aspect of the invention there is provided a method of producing sourdough by fermentation of lactobacilli, that catalyze lactate to propanediol and acetate conversion, the lactobacilli selected from the *Levilactobacillus* species.

### BREIF DESCRIPTION OF THE FIGURES

To easily identify the discussion of any particular element or act, the most significant digit or digits in a reference number refer to the figure number in which that element is first introduced.
FIG. 1 illustrates shows different colony morphologies that were picked and streaked until uniform morphologies were achieved.
FIG. 2 illustrates a typical 50% takeaway and replenishing backslopping technique.
FIG. 3 illustrates a 'Cool/Cold Method' of developing and maintaining a natural stable heterofermentative Lactic Acid Bacteria based sourdough.
FIG. 4 illustrates an alternative 'Cool/Cold Method' of developing and maintaining a natural stable heterofermentative Lactic Acid Bacteria based sourdough.
FIG. 5 illustrates another 'Cool/Cold Method' of developing and maintaining a natural stable heterofermentative Lactic Acid Bacteria based sourdough.
FIG. 6 illustrates yet another 'Cool/Cold Method' of developing and maintaining a natural stable heterofermentative Lactic Acid Bacteria based sourdough.
FIG. 7 illustrates the approximate proportions of microorganisms present in the sourdough pasta and the commercially available non-sourdough pasta.
FIG. 8A to FIG. 8E illustrate microbial composition of an embodiment of the sourdough at the genus level.
FIG. 9A to Fig 9H illustrate the effect of acute intake of conventional and sourdough pasta on, (Fig A) blood glucose, (Fig B) insulin, (Fig C) C-peptide, (Fig D) Glucagon, (Fig E) PYY, (Fig F) GLP-1, (Fig F) GIP and (Fig H) paracetamol.
FIG. 10A and Fig 10B illustrate the effect of chronic intake of conventional and fermented sourdough pasta on (Fig A) blood glucose, (Fig B) fecal SCFA concentration. All data are presented as mean ± SEM (n = 16). ^{∗}Indicates statistical significance in total SCFA concentration (p < 0.05) compared to baseline values.
FIG. 11A - Fig 11F illustrate the impact of chronic consumption of conventional vs. sourdough pasta on bacterial and fungal diversity of overweight participants after consumption of conventional and sourdough pasta for 5 days. (Fig A) Bacterial and (Fig B) fungal abundance; (Fig C) bacterial and (Fig D) fungal beta diversity; (Fig E) bacterial and (Fig F) fungal alpha diversity assessed using Shannon Index.
FIG. 12 illustrates the pathway for anaerobic degradation of lactic acid.

### DETAILED DESCRIPTION

Devices and methods for carrying out the invention are presented in terms of embodiments depicted within the FIGS. However, the invention is not limited to the described embodiments, and a person skilled in the art will appreciate that many other embodiments of the invention are possible without deviating from the basic concept of the invention, and that any such work around will also fall under scope of this invention. It is envisioned that other styles and configurations of the present invention can be easily incorporated into the teachings of the present invention, and the configurations shall be shown and described for purposes of clarity and disclosure and not by way of limitation of scope.

The features of the invention which are believed to be novel are particularly pointed out in the specification. The present invention now will be described more fully hereinafter with reference to the accompanying drawings, which are intended to be read in conjunction with both this summary, the detailed description and any preferred and/or particular embodiments specifically discussed or otherwise disclosed. This invention may, however, be embodied in many different forms and should not be construed as limited to the embodiments set forth herein; rather, these embodiments are provided by way of illustration only and so that this disclosure will be thorough, complete and will fully convey the full scope of the invention to those skilled in the art.

### EXAMPLE 1

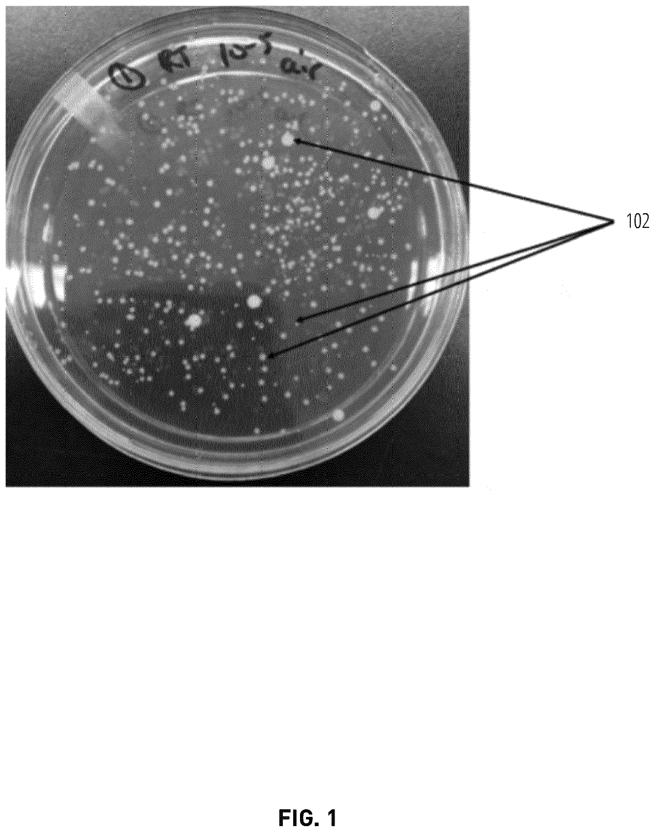

FIG. 1 shows different colony morphologies, 102, that were picked and streaked until uniform morphologies were achieved.

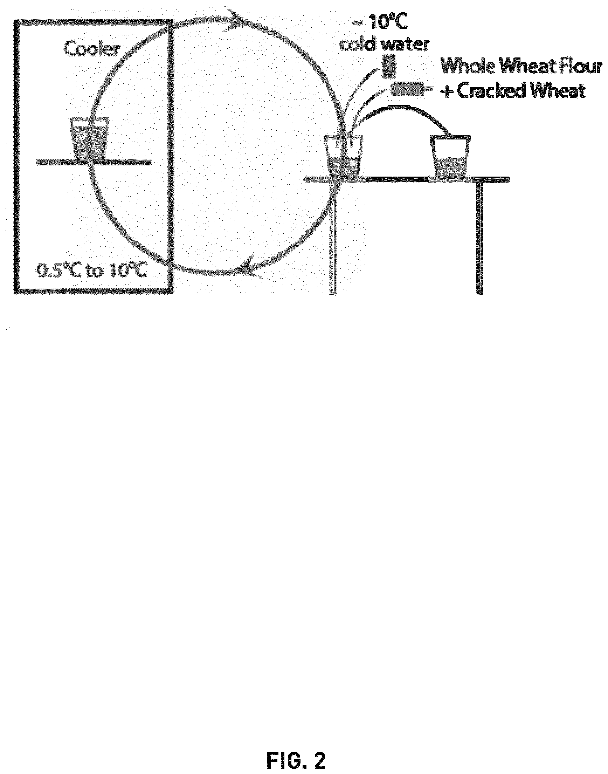

FIG. 2 demonstrates the essence of the maintenance of lactate to acetate + propanediol converting lactobacilli is (i) the high inoculum used for the mother (50% previous sourdough + fresh flour); this keeps the pH low all the time and (ii) the long-term incubation at 4°C. Several publications refer to 4°C as a preservation step that interrupts all microbial activity. Consequently, the presence of lactate to acetate + propanediol converting *Levilactobacillus* is virtually unprecedented. The selection of these organisms as stable components of the sourdough is preferably achieved by a multistage fermentation process at temperatures ranging from 4 °C to 35 °C with backslopping of 5 - 50% of inoculum. One of the stages is preferably conducted at a temperature below ambient for more than 3 days; other stages are preferably conducted at 20 - more than 30 °C.

### EXAMPLE 2

This example outlines methods for the preparation of the sourdough culture and fermented sourdough composition that can subsequently be used in the preparation of pasta.

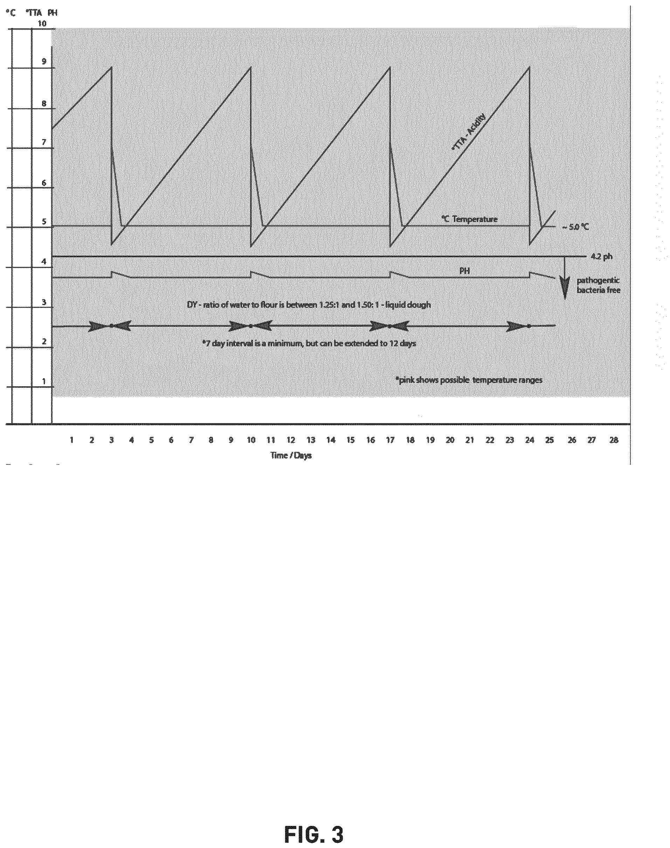

FIGS. 3-7 show examples of 'Cool/Cold Methods' of developing and maintaining a natural stable heterofermentative Lactic Acid Bacteria based sourdough.

The first 'Cool/Cold Method' of developing and maintaining a natural stable heterofermentative Lactic Acid Bacteria based sourdough is illustrated in FIG. 3. This method demonstrates having 7-day intervals with 50% backslopping at 5°C. It is intended that the 7-day backslopping intervals are a minimum, but can be extended to 12 days. The ratio of water to flour is between 1.25:1 and 1.50:1. The pH range is maintained below 4.2.

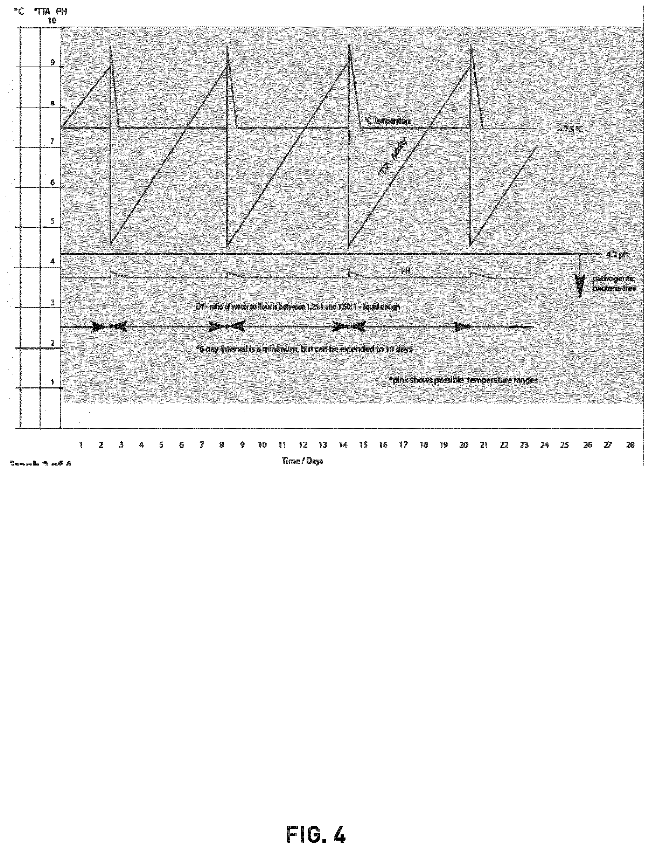

The second 'Cool/Cold Method' of developing and maintaining a natural stable heterofermentative Lactic Acid Bacteria based sourdough is illustrated in FIG. 4. This method demonstrates having 6-day intervals with 50% backslopping at 7.5°C. It is intended that the 6-day backslopping intervals are a minimum, but can be extended to 10 days. The ratio of water to flour is between 1.25:1 and 1.50:1. The pH range is maintained below 4.2.

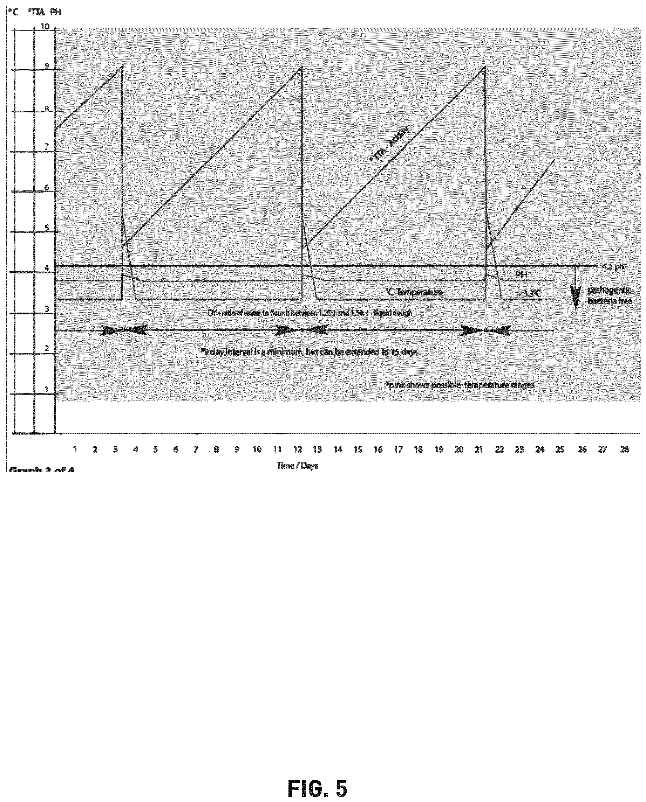

The third 'Cool/Cold Method' of developing and maintaining a natural stable heterofermentative Lactic Acid Bacteria based sourdough is illustrated in FIG. 5. This method demonstrates having 9-day intervals with 50% backslopping at 3.3°C. It is intended that the 9-day backslopping intervals are a minimum, but can be extended to 15 days. The ratio of water to flour is between 1.25:1 and 1.50:1. The pH range is maintained below 4.2.

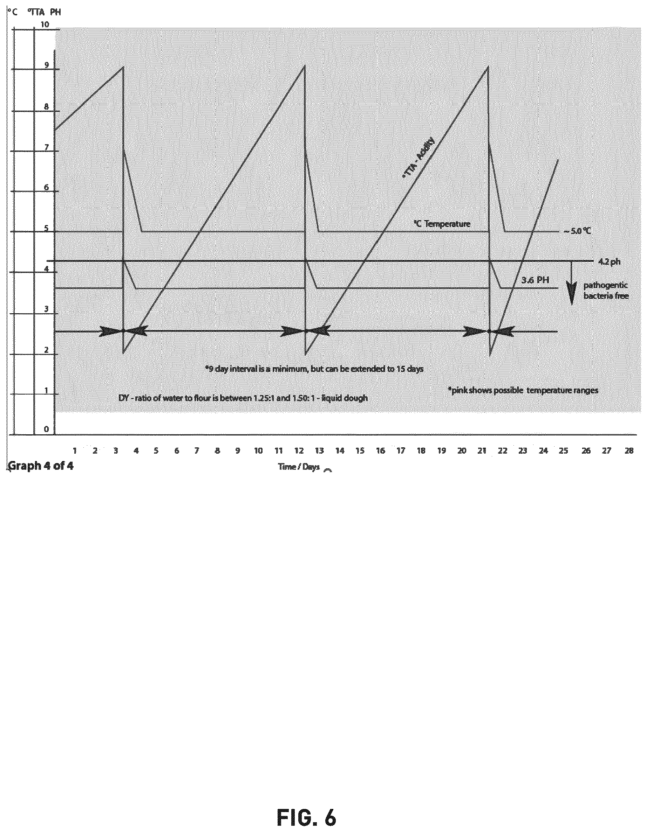

The fourth 'Cool/Cold Method' of developing and maintaining a natural stable heterofermentative Lactic Acid Bacteria based sourdough is illustrated in FIG. 6. This method demonstrates having 9-day intervals with 80-90% backslopping at 5°C. It is intended that the 9-day backslopping intervals are a minimum, but can be extended to 15 days. The ratio of water to flour is between 1.25:1 and 1.50:1. The pH range is maintained below 4.2.

### EXAMPLE 3

This example identifies the composition of microorganisms present in the sourdough used in an embodiment of the cultures used in this unique pasta making process.

### Materials and Methods:

Four sourdough samples were used for culturing and isolation:
a. Sourdough after first stage (fermentation);
b. Sourdough after second stage (ambient);
c. Pasta after mixing, and Pasta after mixing and 24 h of storage at ambient temperature;
d. Pasta after drying.

Samples were obtained and analyzed. In addition. frozen pasta samples (after mixing but before drying) were analyzed after 4 - 5 days of storage at ambient temperature.

**Table 1A Conditions used for bacterial isolation**

| **Media** | **Supplementation** | **Temperature** | **Condition** |
|---|---|---|---|
| **modified MRS** | malt extract | ambient, 30°C | aerobic, anaerobic |
| **(mMRS)** | | | |
| **mMRS** | malt extract, antibiotics | ambient, 30°C | aerobic, anaerobic |
| **mMRS** | Vitamins | ambient, 30°C | aerobic, anaerobic |
| **mMRS** | vitamins, antibiotics | ambient, 30°C | aerobic, anaerobic |
| **M17** | Glucose | ambient, 30°C | aerobic, anaerobic |
| **M17** | glucose, antibiotics | ambient, 30°C | aerobic, anaerobic |

Generally, modified MRS with addition of malt extract or vitamins is sufficient for cultivation of all lactic acid bacteria in sourdoughs; however, to accommodate the presence of unusual bacteria after fermentation at 4 °C, a more extensive set of cultivation media and cultivation conditions was used. The addition of antibiotics was used to selectively identify yeasts.

Mannitol salt agar was used for specific detection of staphylococci in pasta that was frozen after mixing and extrusion, and stored at ambient temperature.

Isolate identification was carried out by first conducting random amplified polymorphic DNA (RAPD) analysis to eliminate clonal isolates followed by 16S sequencing to identify isolates at the species level. Details of culture isolation from food samples are attached in the SOP.

In addition, the ninhydrin method was used to determine the enzymatic activity in the sourdough noodles upon storage at ambient temperatures. Ninhydrin is a chemical which reacts with amines and amino acids in solution producing a colorimetric difference. Extraction of samples was carried out using 100 mM sodium buffer for 1 h at 5°C. The supernatant was combined with the ninhydrin reagent and heated for 16 min followed by measurement at 570 nm.

HPLC analysis of sourdough pasta samples were carried out to determine presence of organic acids during different processing steps. Samples were prepared at a 1:1 ratio with perchloric acid, left overnight at 4 °C, centrifuged and filtered before loading onto the Aminex HPX-87H organic acid column for analysis.

### Results:

The sourdough has a stable core microbiota consisting of *Levilactobacillus koreensis, Lv. parabrevis, Pediococcus parvulus,* and *P. pentoceaus.* The two predominant species *Lv. koreensis* and *P. parvulus* were present at approximately 10⁷ CFU/g to 10⁸ CFU/g in the samples before extrusion. Other bacteria were observed in individual samples or in individual batches, e.g. *Fl. sanfranciscensis* and *Lactococcus lactis.*

### Details are shown in Table 1B for the 3 different batched of sourdough pasta

Table 1B. Bacterial counts and isolates found in different sourdough pasta samples.

**Table 1B - Batch 1**

| | **Batch #1** | | | |
|---|---|---|---|---|
| | **Sourdough 6d fermentation** | **Sourdough ambient** | **Pasta after mixing** | **Pasta after drying** |
| **pH** | **3.61** | **3.53** | **-** | **-** |
| **Total cell count (CFU/g)** | **4-6 × 10⁸** | **4-9 × 10⁸** | **3-9 × 10⁴** | **10²- 10³** |
| I**solates found (^{∗}predomin ant 10⁷ - 10⁸; yeasts ≤ 10⁵) # present only in low cell count (< 1% of total)** | ***Lactobacillus koreensis*^{∗} *Lactobacillus parabrevis Pediococcus parvulus*^{∗} *Pediococcus pentosaceus Pichia membranefaciens Kazachstania barnetti Lactobacillus sanfrαnciscensis Lactococcus lactis*^{∗}** | ***Lactobacillus koreensis*^{∗} *Lactobacillus parabrevis Pediococcus parvulus*^{∗} *Pediococcus pentosaceus Pichia membranefaciens Kazachstania barnetti Lactobacillus sanfranciscensis*** | ***Lactobacillus koreensis*^{∗} *Lactobacillus parabrevis Pediococcus parvulus*^{∗} *Pediococcus pentosaceus Kazachstania barnetti Lactobacillus sanfranciscensis*** | **Lactobacillus *koreensis*^{∗} *Lactobacillus parabrevis Pediococcus parvulus*^{∗} *Pediococcus pentosaceus Lactobacillus sanfranciscensis Staphylococcus warneri*^{∗}** |

**Table 1B - Batch 2**

| **Batch #2** | | | | |
|---|---|---|---|---|
| **Sourdough 6d fermentation** | **Sourdough ambient** | **Pasta after mixing** | **Pasta after mixing 24h** | **Pasta after drying** |
| **3.57** | **3.61** | **-** | **-** | **-** |
| **1-4 × 10⁹** | **10⁷-10⁹** | **6 × 10⁴ - 8 × 10⁵** | **1-6 × 10⁴** | **0-1000** |
| ***Lactobacillus koreensis*^{∗} *Lactobacillus parabrevis Pediococcus parvulus*^{∗} *Pediococcus pentosaceus Pichia membranifaciens Torulaspora delbrueckii Enterococcus lactis^{#}*** | ***Lactobacillus koreensis*^{∗} *Lactobacillus parabrevis Pediococcus parvulus*^{∗} *Pediococcus pentosaceus Pichia membranifaciens Acetobacter cerevisiae*^{∗} *Carnobacterium maltaromaticum*^{∗}** | ***Lactobacillus koreensis*^{∗} *Lactobacillus parabrevis Pediococcus parvulus Pediococcus pentosaceus Kazachstania barnetti*** | ***Lactobacillus koreensis*^{∗} *Lactobacillus parabrevis Pediococcus parvulus Pediococcus pentosaceus Kazachstania barnetti Enterococcus lactis Erwinia ssp*** | ***Lactobacillus koreensis*^{∗} *Lactobacillus parabrevis Pediococcus paravulus*^{∗} *Pediococcus pentosaceus Enterococcus lactis Staphylococcus warneri*** |

**Table 1B - Batch 3**

| **Batch #3** | |
|---|---|
| **Sourdough 6d fermentation** | **Sourdough ambient** |
| **3.62** | **3.7** |
| **9 × 10⁸ - 2 × 10⁹** | **1-4 × 10⁹** |
| ***Lactobacillus koreensis*^{∗} *Lactobacillus parabrevis Pediococcus parvulus*^{∗} *Pediococcus pentosaceus Pichia membranifaciens Kazachstania barnetti*** | ***Lactobacillus koreensis*^{∗} *Lactobacillus parabrevis Pediococcus parvulus*^{∗} *Pediococcus pentosaceus Pichia membranifaciens Kazachstania barnetti Saccharomyces cerevisiae Naumovozyma castellii*** |

Note: *Lactobacillus koreensis* and *Lactobacillus parabrevis* are now assigned to the new genus *Levilactobacillus. Lactobacillus sanfranciscensis* is assigned to the new genus *Fructilactobacillus.*

These organisms represent species that are present in low abundance and were detected only at cultivation conditions that do not support growth of dominant organisms *(Enterococcus lactis, Lactococcus lactis, Carnobacterium maltaromaticum, Acetobacter cerevisiae). Fl. sanfranciscensis* was detected on one batch but not in others. *Fl. sanfranciscensis* is detected only if it account for 1% or more of total bacterial cell counts; results thus indicate that *Fl. sanfranciscensis* is present in low cell counts and occasionally reaches higher cell counts.

Yeast cell counts accounted for less than 0.01% of total microbial counts - this is substantially lower when compared to most other sourdoughs where typically 1 - 10% of microbial cells are yeasts. *Pichia membranifaciens* - a non-fermenting yeast which likely grows only at the surface - and *Kazachstania barnetti* were isolated in all batches of the pasta sourdough.

The presence of *Lv. koreensis* and *P. parvulus* as dominant members of sourdough microbiota and the low yeast cell counts may not be unprecedented but are highly unusual - the exceptional fermentation conditions apparently select for exceptional microorganisms. The high cell counts of *Levilactobacillus* species likely relates to their ability to convert lactate, which support metabolic activity and growth at cold and acidic conditions.

The exceptional presence of *Lv. koreensis* and *Lv. parabrevis* is also reflected by the exceptional profile of metabolites. In particular, the conversion of lactate to acetate and 1,2 propanediol is generally not observed in sourdoughs. Acetic acid is volatile and impacts product flavor more strongly than lactic acid. The levels in pasta dough after mixing - 5 mmol / kg - may be sufficient to impact product flavor.

Lactate conversion to acetate and 1,2 propanediol has been used in experimental sourdoughs to achieve an extended mold free shelf life of bread but is not typically observed in traditional or industrial sourdoughs. One exception pertains to the use of a lactate converting strain of *Lentilactobacillus buchneri* which is used in an industrial application. Here, lactate conversion to acetate and 1,2 propanediol warrants a high metabolic activity of lactic acid bacteria even after extended refrigerated storage.

Extrusion of pasta (samples after pasta mixing) reduced cell counts from one billion cells / g to 10,000 viable cells / g. Major organisms that were identified in sourdough were also identified in pasta after mixing and drying. In addition, enterococci, staphylococci and *Erwinia spp.* were detected, which likely represent contaminants from the processing environment or the flour. Low levels of these organisms are not of concern for food safety.

Analysis of the pasta after drying and days or weeks of dry storage suggested that sourdough lactic acid bacteria die relatively rapidly after drying while plant-associated enterococci and *Enterobacteriaceae* persist in low cell counts.

Storage of frozen pasta for 4 - 5 days at ambient temperature resulted in re-growth of *Pediococcus parvulus* to high cell counts (108 - 109) but not of lactobacilli (see Table 1C).

Table 1C - Bacterial counts and isolates found in extruded pasta noodle samples.

**Table 1C**

| | **Extruded pasta** |
|---|---|
| | Extruded pasta after 4 - 5 days of storage at ambient temperature |
| **pH** | - |
| **Total cell count (CFU/g)** | 8 × 10⁸ - 1 × 10⁹ |
| **Isolates found (*predominant 10⁷ -** 10⁸; **yeasts 10⁴⁻⁷)** | *Pediococcus parvulus*^{∗} *Pichia membranifaciens Kazachstania barnetti* On MSA: *Staphylococcus agenteus*/*aureus* (1×10⁵ CFU/g) *S. warneri* (3×10⁴ CFU/g) *S. saprophyticus* (10³ CFU/g) *S. succinus* (10³ CFU/g) |

Apparently, the water content and water activity in the pasta dough after mixing, estimated at about 30% and 0.9, respectively, was too low to support re-growth of *lactobacilli. Staphylococcus* strains were also present on the noodle, but at counts of approximately 104 - 105 CFU/g.

One of the *Staphylococcus strains* was similar to *Staphylococcus aureus* - although we did not confirm the species identification by re-sequencing, and although the cell counts are close but not quite at a level where toxin production by *S. aureus* presents a safety hazard, this indicates that the post-extrusion fermentation of pasta dough should be controlled to prevent growth of enterotoxin producing staphylococci.

Proteolytic activity during ambient storage of pasta after mixing and freezing was assessed with the ninhydrin assay. The content of primary amines increased more than twofold during storage of pasta (after mixing) for 4 - 5 days, indicating continued activity of cereal proteases. The content of primary amines in pasta after drying was also higher when compared to the sample analyzed after mixing and before drying, however, because these two samples were obtained from different batches, it cannot be concluded with certainty that this represents proteolytic activity during drying of pasta (Table 1D).

Table ID - Ninhydrin assay of frozen pasta noodles and dried pasta samples.

**Table 1D**

| **Ninhydrin Assay** | |
|---|---|
| **Frozen pasta after arrival** | 76.86 ± 14.65 mmol/kg |
| **Frozen pasta after 4-5 d ambient temperature** | 218.06 ± 5.32 mmol/kg |
| **Pasta after drying (from batch #3)** | 110.16 ± 13.32 mmol/kg |

### Summary:

Samples of sourdoughs were obtained at three time points and were shown to contain a core microbiota consisting of *Pediococcus parvulus, Levilactobacillus koreensis, Pediococcus pentosaceus and Levilactobacillus parabrevis.* Yeasts accounted for 0.01% or less of total microbial counts. This composition of sourdough microbiota is exceptional when compared to other sourdoughs - a description of more than 500 sourdoughs is available in the literature - and likely reflects the exceptional fermentation conditions.

Pasta mixing and extrusion reduces bacterial cell counts to less than 10,000 and re-growth of pediococci takes more than 24h. The substantial inactivation of bacterial cells likely represent local temperature inhomogeneities in the extruder - the temperature inactivation of pediococci requires local temperatures of more than 60°C. Bacterial metabolism thus does not occur during drying but flour enzymes may remain active.

Pediococci but not lactobacilli re-grow if pasta dough is stored at ambient temperature after mixing and before drying. Such incubation may require measures to control growth of molds and staphylococci.

Table 1E - Concentration of organic acids and alcohols in sourdoughs, pasta dough, and pasta samples.

**Table 1E**

| | Lactate (mmol / kg) | Acetate (mmol / kg) | 1,2-propanediol (mAU^{∗}min)^{a} | Ethanol (mmol / kg) |
|---|---|---|---|---|
| Batch 1- SD after 6d fermentation | 99.6 ± 2.6 | 125.5 ± 6.7 | 41.92 | 80.4 ± 1.8 |
| Batch 2- SD after 6d fermentation | 84.6 ± 5.3 | 110.7 ± 6.0 | 12.45 | 77.2 ± 7.3 |
| Batch 3- SD after 6d fermentation | 78.1 ± 8.9 | 87.7 ± 0.4 | - | 87.4 ± 0.5 |
| Batch 2- SD after ambient temperature | 30.6 ± 5.3 | 31.3 ± 5.1 | 4.96 | 85.6 ± 17 |
| Batch 3- SD after ambient temperature | 55.3 ± 11 | 39 ± 7.9 | 2.85 | 110.3 ± 11.2 |
| Batch 2- pasta after mixing | 5.7 ± 0.11 | 4.6 ± 0.1 | 1.53 | 0.13 ± 0.01 |

The lactate concentrations in sourdough samples shown in Table 1E match those typically found in sourdoughs, however, the concentrations of acetic acid were 5 - 10 times higher than usually found in sourdough, and acetate concentrations exceeded lactate concentrations after 6 d of fermentation at 4°C. This exceptional high concentration of acetate is attributed to conversion of lactate to acetate and 1,2 propanediol by *Levilactobacillus parabrevis* and/or *Levilactobacillus koreensis.*

The concentration of organic acids in pasta dough after mixing matches the concentration that would be expected by dilution of the sourdough, confirming that lactic acid bacteria show very little metabolic activity after mixing of the pasta dough.

### EXAMPLE 4

This example compares the composition of microorganisms present in the sourdough used in this unique pasta making process with the composition of microorganisms present in a commercially available non-sourdough pasta.

### Materials and Methods:

Two different samples of pasta were used for culturing and isolation:
a. Catelli commercial pasta sample 202; and
b. sourdough pasta sample 204 from the unique pasta making process.

Samples were prepared by homogenizing 2.2g of pasta in 5ml distilled water.

The resulting homogenate was flash frozen at 80⁰C to prevent bacterial growth.

Standard library primers were then used for MLSA typing of the selected genera.

### Results

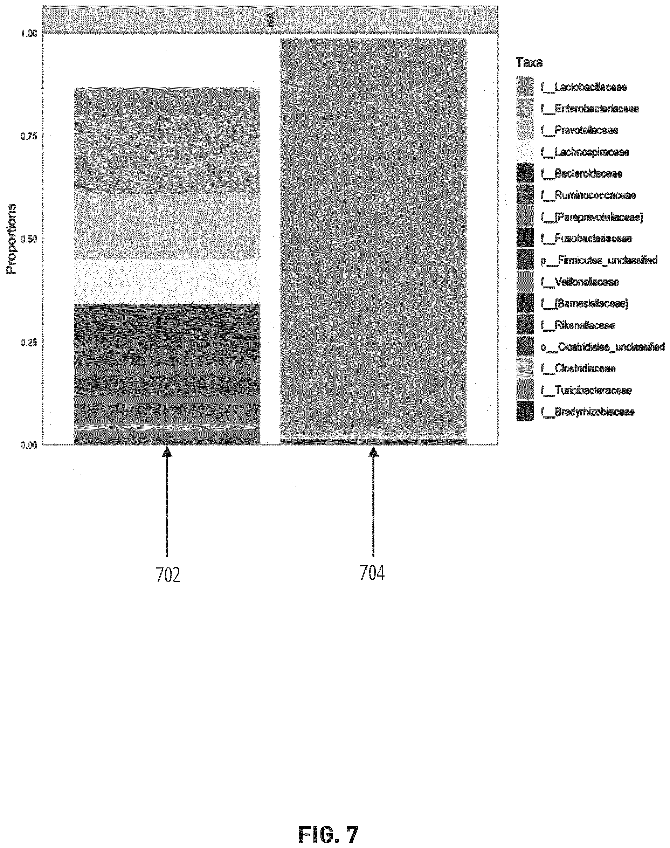

FIG. 7 illustrates the approximate proportions of microorganisms present in the sourdough pasta and the commercially available non-sourdough pasta.

Both pasta products contained bacterial 16S rRNA genes.

### EXAMPLE 5

This example compares microorganisms (specifically 4 genera of the *Lactobacillaceae)* and lactaldehyde dehydrogenase in 11 commercial pastas with 3 samples of sourdough pasta and includes an analysis of dry wheat and dry rye samples.

### Materials and Methods:

The taxonomic identification of bacterial isolates was based on the elimination of clonal isolates by random amplification of polymorphic DNA (RAPD) typing, followed by sequencing of 16S rRNA genes were conducted as outlined in Appl. Environ. Microbiol. 2019, 85 (11), e03130-18.

1. DNA was isolated from the pasta products.

5.0 g of each of pasta product.

Use H2O to soft the pasta (total volume of pasta + H₂O is 50 ml).

Homogenized by the stomacher for 5 min.

Centrifuge at 500 g for 15 min.

Transfer the supernatants into clean tubes and centrifuge at 5000 rpm for 15min.

Harvest cell pellets.

Use the Blood & Tissue Kit to isolate DNA.

2. Primer design for the amplification of genes targeting lactaldehyde dehydrogenase (LAD)

Using lactaldehyde dehydrogenase sequence (KRK67102.1) from *Ln. buchneri* to retrieve protein sequences from *Lactobacillaceae* in NCBI.

Selecting sequences from *Secundilactobacillus* (8 AA sequences), *Levilactobacillus* (6 AA sequences), *Lentilactobacillus* (7 AA sequences), *Furfurilactobacillus* (1 AA sequences), and *Agrilactobacillus* (1 AA sequences).

Importing all selected sequences into Geneious app and aligning them using MUSCLE.

Choosing two conserved amino acid sequences (ADYFDYM; WNFPFF).

ADYFDYM served as the design of forward primer (nucleic acid sequence: 5'-GCNGAYTAYTTYGAYTAYATG-3').

WNFPFF served as the design of reverse primer.

(nucleic acid sequence: 5'-TGGAAYTTYCCNTTYTTY-3'; reverse complementary sequence: 5' -RAARAANGGRAARTTCCA-3 ').

Table 2 - Primers used in Example 5

**Table 2A**

| Targeted genes | Primer (forward, F; reverse, R) | Sequence (5'-3') | Amplicon length (bp) | Reference or source |
|---|---|---|---|---|
| *bacteria* | 27F | AGAGTTTGATCCTGGCTCAG | 1500 | ¹ |
| | 1492R | GGCTACCTTGTTACGACTT | | |
| *Lab* | *Lab,* F | AGCAGTAGGGAATCTTCCA | 341 | ¹ |
| | *Lab,* R | CACCGCTACACATGGAG | | |
| *LAD* | *LAD,* F | GCNGAYTAYTTYGAYTAYATG | 147 | This study |
| | *LAD,* R | RAARAANGGRAARTTCCA | | |

Real time quantitative PCR (qPCR) (SYBR Green dye)

Standard curve Three fragments were amplified using the genome of L. parabrevis as template with three primer pairs, respectively, and purified by PCR cleanup kit. A 10-dilution series of purified PCR products was used to create standard curve (plot Ct vs log copy number).

Table 2B - Mixture preparation for qPCR (reaction volume: 25 µl)

**Table 2B**

| | **Volume (1x, µl)** |
|---|---|
| 2X SYBR Green master mix | 12.5 |
| Forward primer | 2.5 |
| Reverse primer | 2.5 |
| Template DNA | 1 |
| H₂O | 6.5 |

Table 2C - PCR thermal-cycling conditions for quantifying bacteria with 16S rRNA primer

**Table 2C**

| | Temperature (°C) | duration | cycles |
|---|---|---|---|
| AmpliTaq^{®} Fast DNA Polymerase, UP Activation | 95 | 5 min | hold |
| denaturation | 95 | 10 s | 40 cycles |
| annealing | 55 | 30 s | |
| extension | 72 | 60 s | |

Table 2D - PCR thermal-cycling conditions for quantifying *Lactobacillaceae* with Lab primer

| | Temperature (°C) | duration | cycles |
|---|---|---|---|
| AmpliTaq^{®}) Fast DNA Polymerase, UP Activation | 95 | 5 min | hold |
| denaturation | 95 | 10 s | 40 cycles |
| Annealing/extension | 60 | 30 s | |

Table 2E - PCR thermal-cycling conditions for quantifying lactaldehyde dehydrogenase with LAD primer

**Table 2E**

| | Temperature (°C) | duration | cycles |
|---|---|---|---|
| AmpliTaq^{®} Fast DNA Polymerase, UP Activation | 95 | 5 min | hold |
| denaturation | 95 | 10 s | 40 cycles |
| annealing | 50 | 30 s | |
| extension | 72 | 30 s | |

### Results:

**Table 2F - The results of the above described analyses yielded the following results.**

| sample name | | copies / g pasta products | | |
|---|---|---|---|---|
| | | total bacteria | lactobacillaceae (4 families) | lactaldehyde dehydrogenase |
| pasta products | Pasta 1 | 1788273.2 | 642667.5 | 16519.2 |
| | Pasta 2 | 1046.0 | 120.8 | 719.5 |
| | Pasta 3 | 3756.7 | 222.0 | 317.0 |
| | Pasta 4 | 279.0 | 27.3 | 292.9 |
| | Pasta 5 | 6196.2 | 114.7 | 132.1 |
| | Pasta 6 | 348765.2 | 3859.5 | 446.1 |
| | Pasta 7 | 798.0 | 71.4 | 209.0 |
| | Pasta 8 | 1061.6 | 14.1 | 115.4 |
| | Pasta 9 | 875.7 | 25.2 | 144.8 |
| | Pasta 10 | 2156.7 | 76.0 | 857.4 |
| | Pasta 11 | 8353.9 | 696.2 | 431.9 |
| samples in tubes | Pasta 12 | 589945.2 | 361192.9 | 7484.6 |
| | Pasta 12 | 406498.0 | 202110.5 | 2113.0 |
| | Pasta 13 | 538899.1 | 314539.2 | 14520.5 |
| | Wheat (dry) | 2597666.2 | 5288634.6 | 670760.2 |
| | Rye (dry) | 3368521.8 | 141538.6 | 124353.3 |

Pasta 1 - Kaslo sourdough Pasta
Pasta 2 - Catelli Healthy Harvest
Pasta 3 - Catelli Macaroni
Pasta 4 - Western Family Rotini
Pasta 5 - Gold d'Or Spaghetti
Pasta 6 - Lancia Spaghetti
Pasta 7 - Fiorfiore Rigatoni
Pasta 8 - Annie's Homegrown Shells
Pasta 9 - Kraft Dinner Macaroni Shells
Pasta 10 - Barilla Macaroni
Pasta 11 - Catelli Fusilli
Kaslo Sourdough Pasta 1
Kaslo sourdough Pasta 2
Kaslo sourdough Pasta 3
Dried wheat sourdough for pasta making
Dried rye sourdough for pasta making

### Summary:

Quantitative PCR detects bacterial DNA. The results report "gene copies", i.e. the number of DNA molecules with a specific sequence, which corresponds to the number of bacterial cells encoding for that gene. qPCR amplification of dissolved DNA is fairly accurate (not very precise), however, when isolating DNA from sourdough, the yield is not quantitative and how much DNA isolated depends on the organisms that are present in the dough.

The sourdoughs (wheat and rye) had ~ 10⁵ to 10⁶ copies of any bacterial rRNA genes and an equivalent number of 16S rRNA genes of lactobacilli. The gene copy number of the lactaldehyde dehydrogenase gene, which is responsible for the enzyme that converts lactate to acetate and propanediol and unique to the sourdough pasta, ranged from 10 - 90% of numbers for lactobacilli. The numbers are a bit lower than expected - a microbial culture has 10exp9 bacterial cells so a 1000 fold higher count would have been the expectation but the yield of DNA from the sourdough can differ between strains.

From the pasta products, we detected a high (more than 10⁵) count of bacterial DNA in all Kaslo sourdough products and one other. High (more than 10⁵) counts of lactobacillus DNA was found only in the Kaslo products and the gene coding for lactaldehyde dehydrogenase was found only in the Kaslo products. The counts of DNA were lower in the pasta products than in the sourdoughs, which means that DNA is either partially destroyed during drying or is more difficult to isolate.

The data will serve as proof of concept that PCR can be used to detect microbial and enzyme levels in pasta and sourdough pasta and starter cultures.

### EXAMPLE 6

This example demonstrates differences in glycemic content of sourdough and conventional non-fermented pasta.

Increasing consumer interest in fermented products has driven the emergence of a number of novel foods including shelf-stable sourdough pasta. This example comprehensively examined the impact of fermentation on the microbial composition of the culture, pasta, its subsequent effects on glycemic responses and gut microbiota in overweight men and women (>25 kg/m2) compared to a conventional, non-fermented pasta. Two, randomized crossover trials were performed. Study A examined acute feeding responses to each product wherein fasted participants completed a meal tolerance test comprised of 75 g of conventional or sourdough pasta to examine glycemic responses. Study B consisted of three standard oral glucose tolerance tests as well as fecal collection for sequencing at baseline and following each pasta intervention (150 g or 2 serving/d for 5 days) followed by a 2-week washout period.

### Materials and Methods:

The study design was a randomized, double-blind, crossover trial. Participant inclusion criteria included >25 years of age and a body mass index (BMI) > 25 kg/m2 at the time of the study. Researchers initially screened volunteers for overall health status and excluded those with a medical history of gluten allergy, dyslipidemia, gastrointestinal disease, diabetes, other chronic diseases, as well as those taking prescription medications, antibiotics (past 6 months) or natural health products (e.g., already consuming regular pre- or probiotics). Pregnant volunteers and smokers were also excluded from participating.

Table 3 - A CONSORT flow chart of participants enrolled in the study.

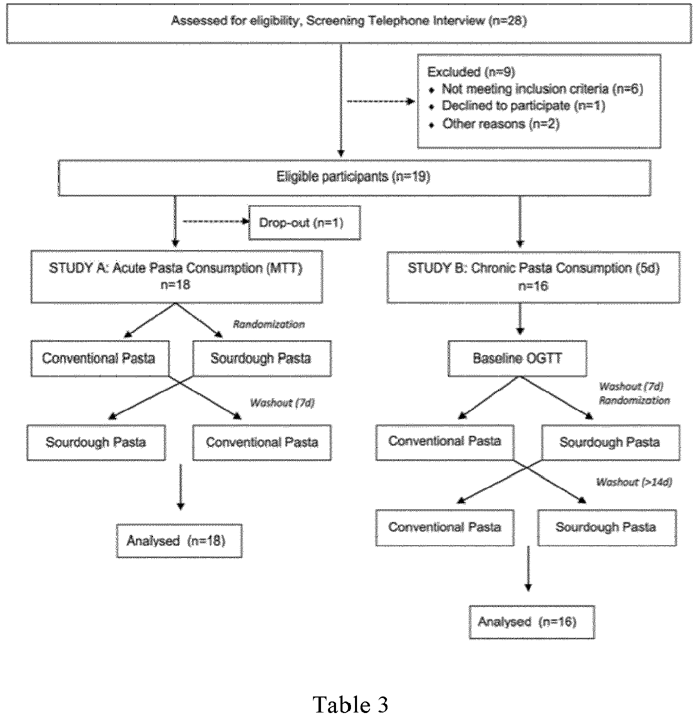

Following informed written consent from eligible volunteers, basic demographic information (e.g., age, sex), anthropometric measures (e.g., height, weight, calculated BMI, waist circumference, hip circumference), and health measures (e.g., resting blood pressure, resting heart rate) were collected. A full-body dual-energy x-ray absorptiometry (DEXA) scan was performed on each participant to analyze body composition.

The sourdough culture is maintained by back-slopping at Kaslo Sourdough (Kaslo, BC, Canada) for the purpose of making bread and pasta. To characterize the microbial composition of the sourdough culture, bacterial and fungal composition was assessed at two time points. The first (SD1) was the starter (mother) culture, while the second renewed (daughter) culture had added flour and water and was mixed before fermentation at ambient temperature for 14-18 h (SD2).

Conventional and Sourdough Pasta Conventional (Catelli R Classic Rotini, Catelli Food Corporation. Winnipeg, MB, Canada) and sourdough pasta (Kaslo Sourdough Pasta Fermentata^{™} Classic Rotini, Kaslo BC, Canada) were tested in the present study. Both are shelf stable, dried pasta products that are widely available in Canada.

Table 4 - The nutritional profile of Kaslo Sourdough Pasta and Catelli Classic Rotini

**Table 4**

| | **Conventional dried pasta** | **Sourdough dried pasta** |
|---|---|---|
| Grams (g) | 75.0 | 75.0 |
| Calories (kcal) | 282.4 | 262.5 |
| Fat (g) | 1.3 | 0.9 |
| Cholesterol (mg) | 0.0 | 0.0 |
| Sodium (mg) | 0.0 | 0.0 |
| Carbohydrate (g) | 56.5 | 52.5 |
| Fiber (g) | 2.6 | 2.8 |
| Sugars (g) | 2.6 | 0.9 |
| Protein (g) | 9.7 | 8.4 |
| Vitamin A (%) | 0.0 | 0.0 |
| Vitamin C (%) | 0.0 | 0.0 |
| Calcium (%) | 1.9 | 0.0 |
| Iron (%) | 9.4 | 20.0 |
| % *represents percent daly value*. | | |

Pastas were compared on a per mass basis (grams) to simulate equivalent consumer consumption. Although not exact in terms of their composition, differences in overall macronutrient composition are minimal. For all studies, pasta was cooked al dente according to manufacturers' instructions. Visually, there was no overt difference between the two pasta products with both having the same color, texture and classic rotini shape.

### Study A: Acute Pasta Consumption

This study investigated gastric emptying, blood glucose and gut peptide responses following pasta consumption. Eligible participants were asked to come into the laboratory to complete two meal tolerance tests. On the day of the appointment, participants arrived at the laboratory following an overnight fast (10-12 h), abstaining from alcohol, caffeine, strenuous physical activity, and analgesic medications for 24-48 h prior. Once screened, an indwelling catheter was placed in the antecubital vein by a trained nurse to facilitate repeated blood sampling. After baseline blood collection (t = 0 min), participants consumed either 75 g dry weight of conventional or sourdough pasta in a randomized order along with 1 g of paracetamol to track gastric emptying. All pasta was served plain and consumed within 6 min. Additional blood samples were collected at 30, 45, 60, 75, 90, 105, 120, 150, and 180 min. A minimum 7-day washout period occurred before the entire procedure was replicated with the other treatment.

### Study B: Chronic Pasta Consumption

This study investigated the impact of chronic pasta consumption over the course of 5 days. This protocol consisted of three standard oral glucose tolerance tests (OGTT) as well as fecal collection at baseline and following each pasta intervention. All participants provided a baseline fecal sample and completed an OGTT (75 g Trutol) following the pre-study procedures outlined in Study A. Blood samples were collected at baseline and at 15, 30, 60, 90, and 120 min. Following baseline sampling, subjects consumed 150 g dry weight/d of either sourdough or conventional pasta for five consecutive days followed by another OGTT (morning of Day 6) and provided an additional fecal sample. After a washout period of at least 2 weeks, but not more than 4 weeks, participants then switched to the other treatment and repeated the protocol.

### Blood Analyses

At each time point, approximately 5 mL of venous blood was collected. Samples were aliquoted into a 2 mL into a K2EDTA plasma vacutainer, 2 mL into a serum vacutainer, and 1 mL into an Eppendorf tube containing EDTA, diprotinin-A (0.034 g/L blood), sigma protease inhibitor (1 g/L blood), and Pefabloc R SC (1 g/L of blood) to preserve analytes. Tubes were centrifuged for 15 min at 4_{°}C. Supernatant was removed from each sample and transferred into a new labeled Eppendorf tube for storage at -80_{°}C until analysis. Blood samples collected in Study A were assessed by a custom Human Metabolic Array [C-peptide, glucagon, gastric inhibitory peptide (GIP), glucagon-like peptide-1 (GLP-1 active), insulin and peptide YY (total)]. A glucose oxidase assay kit used plasma samples to measure plasma glucose concentrations at all time points. Serum paracetamol concentrations, determined using a paracetamol assay kit, were used to track gastric emptying. Several studies have demonstrated paracetamol concentrations to be a valid measure of gastric emptying.

### Pasta Microbial Analyses

Under sterile conditions 2.2 g of cooked pasta was homogenized into 5 mL of purified, distilled laboratory grade water for analysis. For bacterial and fungal sequencing, DNA was then extracted as per the below described protocol. To assess total bacterial and fungi in the pastas, PCR was performed (Mol Autism. (2016) 7:37) using the following primers for bacterial 16S rRNA genes (Forward: TGAAGAATTGATGGAACTCG, Reverse: CATTGTGGTTCACCGTTC, Tm = 63_{°}C) or the internal transcribed spacer region 1 (ITS1) of fungi (Forward GAGGAAGTAAAAGTCGTAACAAGGTTTC, Reverse GTATCGCATTTCGCTGCGTT, Tm = 56_{°}C).

Standard curves were normalized to the copy number of 16SrRNA genes and ITS1 region using reference strain genome size and gene copy number values obtained from the following reference (http://cels.uri.edu/gsc/cndna.html) for both, bacteria and fungi groups. Data are expressed as gene copies/g pasta water slurry described above. It should be recognized that bacteria and fungi in each pasta are not viable, and that the above analysis was done to highlight differences between the two pastas resulting from the fermentation process. Sourdough samples SD1 and SD2 as well as conventional and sourdough pasta were additionally characterized by high throughput sequencing of 16S rRNA and ITS2 sequence tags as outlined below.

### Fecal Microbial Analyses

Following collection, fecal samples were stored at -80_{°}C. DNA was extracted by a PowerMag Soil DNA Isolation kit (MP Biomedical) as per protocol. 16S rRNA genes and ITS2 regions were targeted for bacterial and fungal sequencing, respectively. PCR amplification was done with dual-barcoded primers targeting the V4 region and sequenced with an IIlumina MiSeq using the 300-bp paired-end kit. Primers and PCR conditions used for 16S sequencing were followed as described *(*Appl Environ Microbiol. (2013) 79:5112-20); those used for ITS2 sequencing were identical to Gweon et al. (Methods Ecol Evol. (2015) 6:973-80). Sequences were denoised, taxonomically classified using Greengenes (v. 13_8) as the reference database, and clustered into 97%-similarity operational taxonomic units (OTUs) with the mothur (Appl Environ Microbiol. (2009) 75:7537-41). Paired end reads were merged and curated to reduce sequencing error. The processing pipeline for investigating fungal populations was identical to the one used for bacteria, except for the following differences: (1) paired-end reads were trimmed at the nonoverlapping ends, and (2) high quality reads were classified using UNITE (v. 7.1) as the reference database. The resulting dataset had 23008 OTUs (including those occurring once with a count of 1, or singletons). An average of 28163 quality-filtered reads were generated per sample. Sequencing quality for R1 and R2 was determined using FastQC 0.11.5. A consensus taxonomy for each OTU was obtained and the OTU abundances were then aggregated into genera with modifications, accounting for the current taxonomy of lactobacilli *(*Int J Syst Evol Microbiol. (2020) 70:2782-858). The datasets generated for this study can be found in NCBI BioProject, NCBI Accession No. PRJNA668386. Fecal Short Chain Fatty Acid Analyses For SCFA of fecal samples, methods followed those previously described (J Nutr Biochem. (2019) 64:228- 36). Briefly, 200 mg of sample was weighed and internal standard (2-ethylbutyric acid) was added. Samples were dissolved in 95% ethanol and homogenized for two cycles. The samples were stored overnight in -80_{°}C and SCFA were derivatized to its hydrazide forms for further analysis using High Performance Liquid Chromatography (HPLC) as described previously with some modifications *(*Ann Clin Biochem. (2010) 47:447- 52). An aliquot of 20 µL of sample was injected into the HPLC. The analytes were separated with the use of a C18 column (150 mm × 4.6 and 2.6 um pore size) at 40_{°}C temperature. A gradient method was applied by using acetonitrile and water as solvents at a flowrate of 0.8 mL/min. SCFA were detected at a wavelength of 230 nm and corresponding peaks were integrated using the OpenLab software from Agilent Technologies.

### Statistical Analysis

Participant data are presented as mean ± SD. All the other data are presented as mean ± SEM unless otherwise indicated. Normality of the data was assessed using the Shapiro-Wilk normality test. Differences between treatments were determined using two-way repeated measures ANOVA and Tukey's multiple comparison post-hoc test for repeated measures for timed data. Area under the curve (AUC) was calculated using the trapezoid method. Statistical analysis was performed using GraphPad Prism (v. 7.0; La Jolla, USA) and the mothur software package. Alpha diversity for bacterial and fungal reports was estimated with the Shannon index on raw OTU abundance tables after filtering out contaminants. The significance of diversity differences was tested with an ANOVA. To estimate beta diversity across samples, we excluded OTUs occurring with a count of less than three in at least 10% of the samples and then abundance-weighted sample pair-wise differences were computed using Bray-Curtis dissimilarity. Beta diversity was visualized using Principal Coordinates Analysis (PCoA) ordination. Variation in community structure was assessed with permutational multivariate analyses of variance (PERMANOVA) with treatment group as the main fixed factor and using 4,999 permutations for significance testing. All analyses were conducted in the R environment. Statistical significance was set at p < 0.05.

### Results

Culture-Independent Analyses of Sourdoughs Sourdough cultures are extremely complex, and vary in their composition. They can contain two to five of more than 100 different species of lactic acid bacteria and one to three of more than two dozen species of yeasts. For this reason, the microbial composition of the culture used in this study is reported. To determine the microbial composition of the sourdough culture, sequencing was performed.

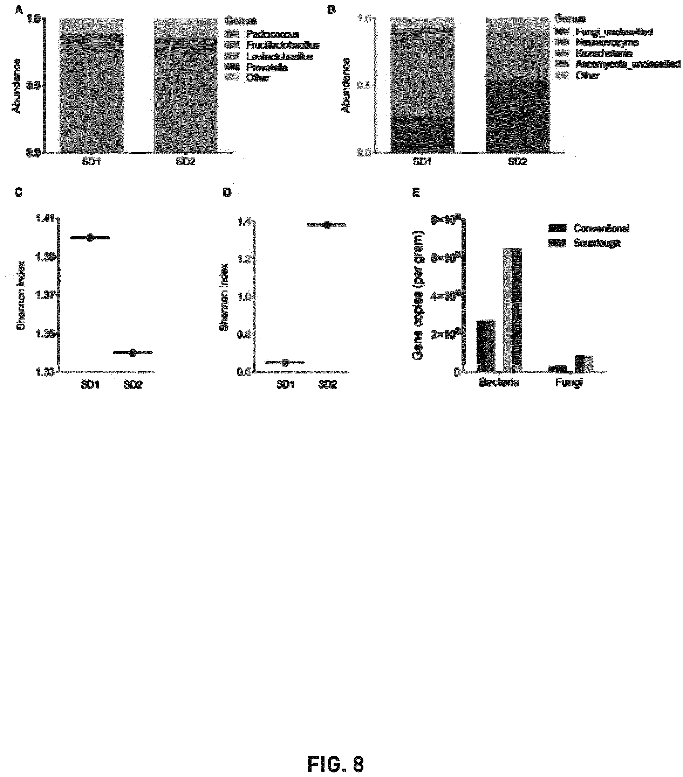

FIG. 8A to Fig 8E - Microbial composition of the sourdough. Results are reported at the genus level. The mother culture (SD1) was dominated by *Pediococcus* (15%) and *Levilactobacillus* (74%); most sequences matched most closely to *Levilactobacillus parabrevis* (Figure 8A). In the second sourdough stage after 14-18 h of fermentation at ambient temperature (SD2), the abundance of *Pediococcus* remained unchanged, *Levilactobacillus* were reduced to 40% and sequences matching the genus *Fructilactobacillus* increased (41%). There was bacterial alpha diversity in SD1 and SD2 remained essentially unchanged (Figure 8B). Fungal analysis showed the SD1 culture to be dominated by *Naumovozyma* genus with *Naumovozyma castelli* (anamorph: *Saccharomyces castelii)* accounting for 55% of abundance. Levels of the *Naumovozyma* genus were reduced in SD2 with growth of *Kazachstania barneti* (26.9%) and other unclassified fungi (53.7%) (Figure 8C). Overall, fungal alpha diversity increased in SD2 (Figure 8D).

To further distinguish the two pasta products and highlight microbial changes taking place with fermentation, the microbial composition of each pasta was assessed. However, it must be realized that the microbes in each were no longer viable. Conventional pasta was dominated by *Prevotella* (15%) while sequences representing the genera *Pediococcus* (64%) and *Levilactobacillus* (28%) were the most prevalent species in the sourdough pasta. Together, these two genera accounted for 95% of the sequences from sourdough pasta. Relative to bacteria, both pasta products displayed minimal fungal diversity with most being unclassified. Analysis of total bacterial and fungal content in the samples showed the sourdough pasta to contain greater counts of bacteria and fungi compared to the conventional product (Figure 8E).

Table 5 - Participant Characteristics

**Table 5**

| | **Study A** | **Study B** |
|---|---|---|
| **Total participants,** n | 18 | 16 |
| Age (years) | 38.6 ± 10.6 | 42 ± 10 |
| Males/females | 6/11 | 5/11 |
| BMI, kg/m² | 29.6 ± 1.0 | 28.46 ± 2.8 |
| Waist circumference, cm | 95.7 ± 3.1 | 93.71 ± 9.1 |
| Hip circumference, cm | 108.9 ± 2.1 | 106.13 ± 7.2 |
| Systolic BP, mmHg | 124.5 ± 3.7 | 117.87 ± 14.9 |
| Diastolic BP, mmHg | 72.3 ± 1.9 | 68.37 ± 9.3 |
| Resting HR, bpm | 68.9 ± 2.2 | 63.93 ± 8.3 |
| Total BMD, g/cm² | 1.14 ± 0.0 | 1.16 ± 0.1 |
| % Fat arm | 34.5 ± 9.9 | 31.60 ± 10.8 |
| % Fat trunk | 32.4 ± 7.6 | 28.21 ± 8.4 |
| % Fat leg | 33.0 ± 11.2 | 31.69 ± 9.9 |
| Total % fat | 32.1 ± 2.0 | 29.29 ± 8.2 |
| *Values represent as mean* ± *SD. BMD, bone mineral density; BMI, body mass index; BP, blood* pressure; *HR, heart rate.* | | |

Study A consisted of 18 participants while Study B consisted of 16 of the same participants. The age and cardiometabolic parameters (BMI, Waist/Hip circumference, resting blood pressure, heart rate) and DEXA scan results were also similar in both, Study A and Study B participants (p > 0.05).

### Study A - Acute Pasta Consumption

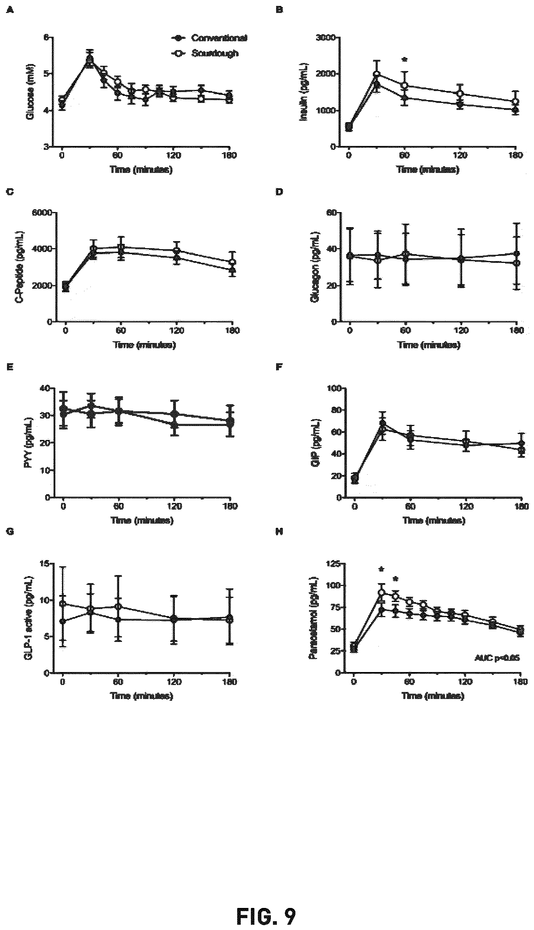

FIG. 9A to Fig 9H illustrate the effect of acute intake of conventional and sourdough pasta on, (Fig A) blood glucose, (Fig B) insulin, (Fig C) C-peptide, (Fig D) Glucagon, (Fig E) PYY, (Fig F) GLP-1, (Fig F) GIP and (Fig H) paracetamol. All data are presented as mean ± SEM (n = 18). Abbreviations: GIP - gastric inhibitory polypeptide, GLP1 - glucagon-like peptide, PYY - peptide YY. ^{∗}Indicates statistical significance (p < 0.05) compared to conventional pasta treatment at a corresponding time point.

To assess the impact of acute pasta consumption, each participant consumed 75 g of conventional or sourdough pasta, followed by assessment of their glycemic response. No differences in baseline glucose or insulin levels were present prior to meal tolerance tests between conventional or sourdough pasta administration (p > 0.05). Following pasta administration, there were no significant differences in the overall responses (total AUC) of glucose (Figure 9A, p > 0.05) and insulin (Figure 9B, p > 0.05). However, insulin levels were greater with sourdough at 60 min post-consumption (p < 0.05). No differences between conventional or sourdough pasta consumption for gut peptide responses including C-Peptide, glucagon, PYY, GIP or GLP-1 were found (Figures 9C-9G, p > 0.05). The total serum paracetamol levels (AUC), a proxy measure of gastric emptying was elevated with sourdough consumption (Figure 9H, p < 0.05).

### Study B - Chronic Pasta Consumption

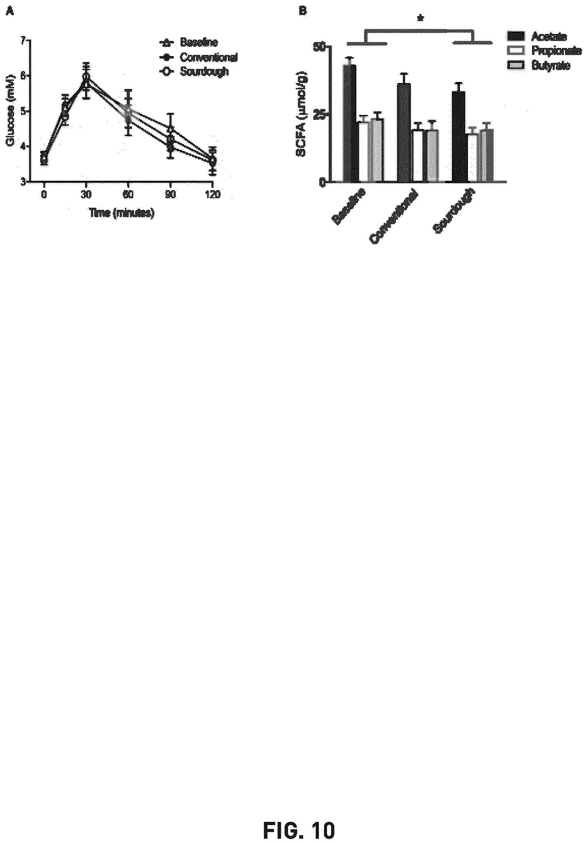

FIG. 10A and Fig 10B illustrate the effect of chronic intake of conventional and fermented sourdough pasta on (FigA) blood glucose, (Fig B) fecal SCFA concentration. All data are presented as mean ± SEM (n = 16). ^{∗}Indicates statistical significance in total SCFA concentration (p < 0.05) compared to baseline values.

To assess the impact of chronic pasta consumption, subjects completed a baseline OGTT followed by 5 days of pasta consumption (150 g/day) in a cross-over design. Similar to previous study A, there was no difference in blood glucose levels prior to OGTT administration in any trial. Likewise, there were no differences in glucose or insulin excursion between baseline (no pasta), conventional and sourdough pasta (Figure 10A, p > 0.05). As the benefits of fermented foods may involve the gut microbiota, microbial metabolites including SCFA (acetate, propionate, butyrate) were analyzed in fecal samples. There was no difference in the concentration of individual fecal SCFA at baseline or with each pasta treatment (Figure 10B, p > 0.05). However, the concentration of total SCFA concentration decreased in the sourdough treatment compared to baseline values (p < 0.05).

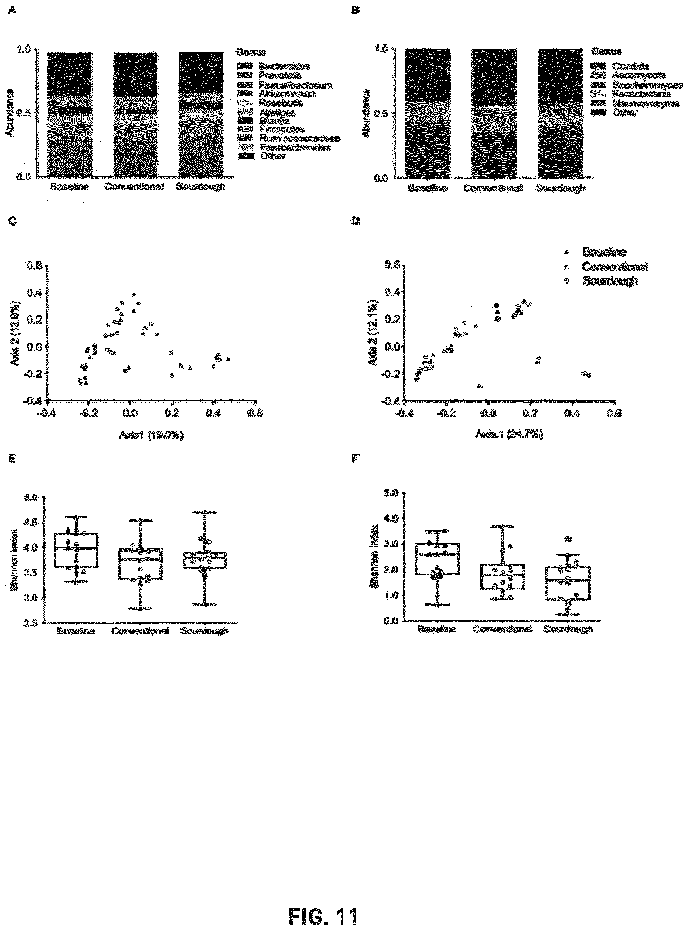

FIG. 11A - Fig 11F illustrate the impact of chronic consumption of conventional vs. sourdough pasta on bacterial and fungal diversity of overweight participants after consumption of conventional and sourdough pasta for 5 days. (Fig A) Bacterial and (Fig B) fungal abundance; (Fig C) bacterial and (Fig D) fungal beta diversity; (Fig E) bacterial and (Fig F) fungal alpha diversity assessed using Shannon Index. ^{∗}Indicates statistical significance (p < 0.05) compared to baseline values.

To investigate the potential impact of pasta consumption on the gut microbiota, both 16S and ITS2 sequencing were performed. No treatment differences were observed for bacterial (Figure 11A) and fungi (Figure 11B) at the phyla, class, family or genus levels (p > 0.05). We next assessed the alpha and beta-diversity as a reflection of how each pasta treatment changed microbial structure (Figures 11C-F). There were no changes found in alpha diversity (F = 1.88, P = 0.17) as assessed by the Shannon Index (Figure 11C, p > 0.05) of bacterial groups with treatment; however, there was a significant decrease in alpha diversity in fungi (F = 5.37, P = 0.008) with chronic consumption of sourdough (Figure 11D, p < 0.05). These differences in microbial structure are also reflected in the separation between groups when plotted as principal coordinates (Figures 11E and 11F).

### Summary

Sourdough fermentation has the potential to increase nutrient bioavailability, decrease the glycemic index and gluten content of foods along with improving product shelf life (20). In the present study, we examined the impact of fermentation on the microbial composition of pasta as well as the acute and chronic effects of consumption on glucose regulation and gut microbial composition in overweight and obese adults. Major findings indicate that acute or chronic consumption of sourdough pasta had no effect on glycemia and other gut hormones in participants compared to conventional, non-fermented pasta. Of interest, we found that the microbiome was adaptive following chronic pasta consumption with reductions in fecal SCFA and decreases in the alpha diversity of fungi following sourdough, but not conventional pasta.

It was found that glucose and insulin responses remain unaltered in overweight individuals after acute and chronic sourdough pasta consumption. We also found that there are no significant changes in gut microbial profile or their associated metabolites after 5 days of dietary intervention. Unexpectedly, consumption of the sourdough product reduced fecal fungal alpha diversity.

### CONCLUSION

Production of food and food ingredients via microbial fermentation is characterized by and intrinsic complexity. The raw material and the final product typically have a complex composition: fermented food is often the result of the metabolic activity of mixed microbial populations, resulting in the production of complex ensemble of primary and secondary metabolites that are responsible for the main and specific features of a certain food or beverage

It had previously been stated [in Microbial production of food ingredients, enzymes and nutraceuticals. Edited by Brian McNeil, David Archer, Ioannis Giavasis and Linda Harvey, Woodhead Publishing, 2013] that "Production of food and food ingredients via microbial fermentation is characterized by and intrinsic complexity. The raw material and the final product typically have a complex composition: fermented food is often the result of the metabolic activity of mixed microbial populations, resulting in the production of complex ensemble of primary and secondary metabolites that are responsible for the main and specific features of a certain food or beverage."

The findings presented herein challenge the general assumption in the science of sourdough fermentation perceives that refrigerated cold temperature of 4°C is only for preservation and that all microbial activity stops. This is disproven with this new 'Cold back-slopping method' for sourdough method. Over an extended period of time of 'cold/cool back-slopping' techniques using distinctive LAB species develop that have or do adapt themselves to the colder environment and start to thrive, and can be utilized for products made with natural sourdough ferment.

As Charles Ellery Avery First Manufacturer of Lactic Acid, in 1881 noted: 'Avery's ideas about preparing pure cultures by selecting conditions favoring the growth of the desired culture while at the same time suppressing the growth of other microorganisms can be seen as an extension of Pasteur who already in his early investigations of lactic and alcohol fermentations had stressed the point that the conditions of the medium play a great part in the predominant development of any given ferment (Nicolle, 1961).' Also, noteworthy is Avery's observation that the inoculation of each solution from the preceding one should be affected at the point of full height of fermentation, as evidenced by the evolution of the carbonic acid gas (i.e. backslopping) Avery, 1883. Page 91. (back-slopping).

Critical observations that enable embodiments of the invention include:
discovering that indeed certain LAB species are capable to adapting to cold environmental conditions;
using 'back-slopping' in combination with the logarithmic nature of the pH scale' thereby ensuring 'natural pure culture' propagation preserving LAB species;
discovering that these LAB are suitable for a multitude of wheat grain-based products like breads, sweet dough, pasta etc. Virtually any product made from gluten and none gluten combinations;
observing that these concentrated LAB produced achieve dominance over other microorganism naturally present in these grain-based products, depressing questionable or pathogenic microorganism naturally present in any cereal based agricultural product. Similarly observed in latest scientific research on the human digestive system, where beneficial LAB crowd out pathogenic microorganism in the human digestive tract. It seems that it becomes a question of momentum, concentration of LAB in order to establish dominance, may it be in products made or the human digestive system;
multitude of testimonials over 30 years verify that product made from these LAB, as with all-natural sourdough ferments, aid in digesting cereal-based crops, reduce allergic reaction and possible avoid gluten sensitivity, irritable bowel symptoms etc; and
recognizing the 4.2ph threshold to advantage though targeted observation and utilizing bacteria growth momentum to ensure if above the time is kept to a minimum to reduce any pathogenic growth. Below pH 4.2 is the threshold where no pathogenic bacterial growth is observed in nature.

Lactobacilli that catalyze the lactate to propanediol and acetate conversion include *Loigolactobacillus coryniformis, Furfurilactobacillus* species, few of the *Levilactobacillus* species and several *Lentilactobacillus* species. Of these, *Furfurilactobacillus* and *Levilactobacillus* species are more or less common in sourdough but the lactate converting levilactobacilli species are not common in sourdough, the conversion has not been shown experimentally for furfurilactobacilli, and the conversion in sourdough has been shown only once in sourdough (Food Microbiol. (2010) 27:390-395) and the species used in that study to convert lactate to acetate and propanediol *(Lentilactobacillus buchneri)* was not a sourdough isolate, does not commonly occur in sourdough, and is not found in any embodiments of the instantly disclosed sourdough.

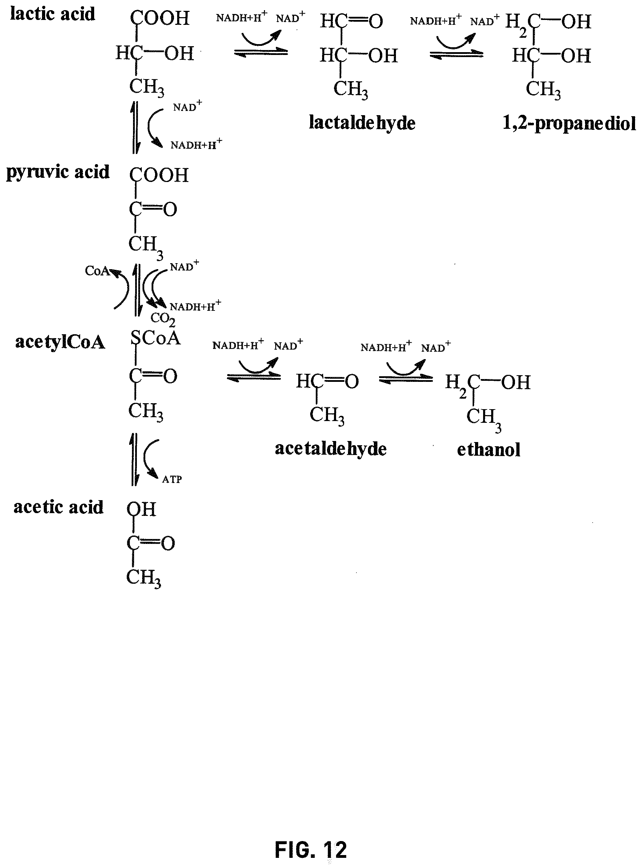

The conversion of lactic acid to propanediol and acetic acid has to date only been observed in *Lentilactobacillus* species, mainly *Lentilactobacillus buchneri.* See Fig 12 for the proposed pathway for anaerobic degradation of lactic acid by *L. buchneri* into equimolar amounts of 1,2-propanediol and acetic acid and trace amounts of ethanol. It is known to be relevant in silage fermentations, and during spoilage of pickled cucumbers. The key gene coding for the conversion has been identified in a few other lactobacilli including few *Levilactobacillus* and *Furfurilactobacillus* species but confirmation that the pathway is functional is missing for *Furfurilactobacillus.*

The conversion has been observed in sourdoughs that are inoculated with *Lentilactobacillus buchneri* (Food Microbiol (2010) 27:390-395 and CA 2759532). The disclosures essentially relate to the use of *Lentilactobacillus buchneri* and lists a number of species but not *Levilactobacillus parabrevis* (which would then have been termed *Lactobacillus) brevis* and *Levilactobacillus koreensis.*

The presence of *Levilactobacillus koreensis* and *Levilactobacillus parabrevis* in the disclosed sourdough is not unprecedented but exceptional. Of the 1,364 sourdoughs for which literature data is available, less than 30 include one or the other (eLife (2021) 10:e61644 and Int J Food Microbiol 239:35-43). None of the two reports indicate the lactate to propanediol conversion.

The present disclosure confirms the novel identification of specific fermentation conditions that reliably maintain lactate-utilizing lactobacilli as dominant members of the microbiota by traditional methods (i.e. without inoculating controlled strains).

Also note that none of the cited prior art documents are connected specific fermentation conditions that catalyze lactate to acetate and propanediol in sourdough pasta making. In sourdough baking, most of the acetic acid in bread is formed in the bread dough, i.e. is less than 3 h and the slow lactate to acetate conversion by *Lentilactobacillus* or *Levilactobacillus* species has no role to play. In pasta making, dough mixing and extrusion kills most bacteria and acetic acid in the final product is produced in the (long and cold) sourdough fermentation.

Numerous cited documents refer to putting a sourdough in the fridge but the sourdoughs do not fermentation in the cold storage. This is not the case for embodiments of the present process. Part of the fermentation process occurs in cold storage. The presence of these unusual combination of *Levilactobacillus koreensis* and *Levilactobacillus parabrevis* almost certainly relates to the cool fermentation. These microorganisms are seen in high proportion after the cold fermentation step and in a much lower proportion of the fermentation after the second fermentation step which happens at anywhere between 20 and 30 degrees centigrade. The reason being that *Levilactobacillus* species to grow in the fridge but *Lentilactobacillus* species do not.

A crucial difference between known processes and the inventive process is that the conditions of the inventive fermentation process naturally select for several species of microorganisms, including *Levilactobacillus koreensis* and *Levilactobacillus parabrevis.* Specifically, the cold fermentation favors growth of these organisms and thereby produces more acetic acid than lactic acid in the sourdough.

The foregoing descriptions of specific embodiments of the present invention have been presented for purposes of illustration and description. They are not intended to be exhaustive or to limit the invention and method of use to the precise forms disclosed. Many modifications and variations are possible in light of the above teaching. The embodiments described were chosen and described in order to best explain the principles of the invention and its practical application, and to thereby enable others skilled in the art to best utilize the invention and various embodiments with various modifications as are suited to the particular use contemplated. It is understood that various omissions or substitutions of equivalents are contemplated as circumstance may suggest or render expedient, but is intended to cover the application or implementation without departing from the spirit or scope of the claims of the present invention.

An aspect of the present invention will now be described by way of reference to the following clauses:
1. A composition for producing a sourdough, the composition comprising one or more lactobacilli, that catalyze lactate to propanediol and acetate conversion, the lactobacilli selected from the *Levilactobacillus* species.
2. The composition of clause 1, wherein the one or more lactobacilli comprises *Levilactobacillus parabrevis.*
3. The composition of clause 1 or clause 2, wherein the one or more lactobacilli comprises *Levilactobacillus koreensis.*
4. The composition of any one of the preceding clauses, wherein the one or more lactobacilli comprise *Levilactobacillus parabrevis* and *Levilactobacillus koreensis.*
5. The composition of any one of the preceding clauses, further comprising one or more microorganisms selected from the group comprising: *Furfurilactobacillus, Lentilactobacillus* and *Secundilactobacillus.*
6. A method of producing sourdough by fermentation of lactobacilli, that catalyze lactate to propanediol and acetate conversion, the lactobacilli selected from the *Levilactobacillus* species.
7. The method of clause 6, wherein the one or more lactobacilli comprises *Levilactobacillus parabrevis.*
8. The method of clause 6 or clause 7, wherein the one or more lactobacilli comprises *Levilactobacillus koreensis.*
9. The method of any one of clauses 6 to 8, wherein the one or more lactobacilli comprise *Levilactobacillus parabrevis* and *Levilactobacillus koreensis.*
10. The method of any one of clauses 6 to 9, wherein the fermentation includes backslopping at 6-9 day intervals.
11. The method of clause 10, wherein the backslopping is conducted at a ratio of water to flour of between 1.25:1 and 1.50:1.
12. The method of any one of clauses 6 to 11, wherein the fermentation is conducted at a pH of below 4.2 for 7-12 days at 5.0°C, with 50% backslopping.
13. The method of any one of clauses 6 to 12, wherein the fermentation is conducted at a pH of below 4.2 for 6-10 days at 7.5°C, with 50% backslopping.
14. The method of any one of clauses 6 to 13, wherein the fermentation is conducted at a pH of below 4.2 for 9-15 days at 3.3°C, with 50% backslopping.
15. The method of any one of clauses 6 to 14, wherein the fermentation is conducted at a pH of below 4.2 for 9-15 days at 5.0°C, with 80-90% backslopping.

## Claims

1. A composition for producing a sourdough, the composition comprising one or more lactobacilli, that catalyze lactate to propanediol and acetate conversion, the lactobacilli selected from the *Levilactobacillus* species.

2. The composition of claim 1, wherein the one or more lactobacilli comprises *Levilactobacillus parabrevis.*

3. The composition of claim 1, wherein the one or more lactobacilli comprises *Levilactobacillus koreensis.*

4. The composition of claim 1, wherein the one or more lactobacilli comprise *Levilactobacillus parabrevis* and *Levilactobacillus koreensis.*

5. The composition of claim 1, further comprising one or more microorganisms selected from the group comprising: *Furfurilactobacillus, Lentilactobacillus* and *Secundilactobacillus.*

6. A method of producing sourdough by fermentation of lactobacilli, that catalyze lactate to propanediol and acetate conversion, the lactobacilli selected from the *Levilactobacillus* species.

7. The method of claim 6, wherein the one or more lactobacilli comprises *Levilactobacillus parabrevis.*

8. The method of claim 6, wherein the one or more lactobacilli comprises *Levilactobacillus koreensis.*

9. The method of claim 6, wherein the one or more lactobacilli comprise *Levilactobacillus parabrevis* and *Levilactobacillus koreensis.*

10. The method of claim 6, wherein the fermentation includes backslopping at 6-9 day intervals.

11. The method of claim 10, wherein the backslopping is conducted at a ratio of water to flour of between 1.25:1 and 1.50:1.

12. The method of claim 11, wherein the fermentation is conducted at a pH of below 4.2 for 7-12 days at 5.0°C, with 50% backslopping.

13. The method of claim 11, wherein the fermentation is conducted at a pH of below 4.2 for 6-10 days at 7.5°C, with 50% backslopping.

14. The method of claim 11, wherein the fermentation is conducted at a pH of below 4.2 for 9-15 days at 3.3°C, with 50% backslopping.

15. The method of claim 11, wherein the fermentation is conducted at a pH of below 4.2 for 9-15 days at 5.0°C, with 80-90% backslopping.
